# EUROPEAN PATENT APPLICATION

(11) **EP 2 113 250 A1**
(43) Date of publication of application: **04.11.2009**
(21) Application number: 09001261.8
(22) Date of filing: 29.01.2009
(51) Int. Cl.: A61K 9/70

(54) **Patch package structure**

(30) Priority: 06.02.2008 JP 2008026631
(71) Applicant: Nitto Denko Corporation, Ibaraki-shi, Osaka 567-8680 (JP)
(72) Inventor: Okada, Katsuhiro, Ibaraki-shi Osaka 567-8680 (JP); Iwao, Yoshihiro, Ibaraki-shi Osaka 567-8680 (JP); Matsuoka, Kensuke, Ibaraki-shi Osaka 567-8680 (JP)
(74) Representative: Grünecker, Kinkeldey, Stockmair & Schwanhäusser Anwaltssozietät

(57) **Abstract**

The present invention provides a patch package structure which includes a package and a patch disposed in the package, in which the package includes a first sheet material which is substantially planar and a second sheet material which has been molded, the second sheet material has a first region which projects outward from the package and a second region which surrounds the first region and is substantially planar, the first region has a planar outer shape which includes a planar outer shape of the patch, and the second region has a first sealed region which surrounds the first region and an unsealed region located nearer to a central part of the second sheet material than the first sealed region. According to the invention, the patch is inhibited from adhering to the inner surface of the package and the package can be easily opened by hand.

## Description

### FIELD OF THE INVENTION

The present invention relates to a patch package structure including a package and a patch disposed in the package.

### BACKGROUND OF THE INVENTION

Patches to be applied to the skin for the purpose of protecting the affected part and patch preparations to be applied to the skin of a mammal for the purpose of percutaneously administering a drug to the mammal have hitherto been developed. Such patches or patch preparations (hereinafter inclusively referred to as patches unless otherwise indicated) are being sold on the market generally in the form of a package structure including the patch disposed in a bag-form package (see, for example, JP-UM-B-4-51782). Such a bag-form package is generally made of a flexible resin film or the like and, hence, it is not difficult to open the bag-form package by tearing it by hand.

However, use of such a bag-form package has the following drawback. The patch is in close contact with the package, and a load is apt to be imposed on the patch from outside the package. Because of this, a component of the pressure-sensitive adhesive layer is apt to leak out through or protrude from a peripheral or other part of the patch, whereby the patch adheres to the inner surface of the package. The patch hence tends to be difficult to take out of the package.

JP-T-10-511330 (the term "JP-T" as used herein means a published Japanese translation of a PCT patent application) discloses a patch package structure including a package having a given container shape and a patch disposed therein. The patch disposed in such a package is less apt to receive a load from outside the package as compared with the patch in a bag-form package. This patch package structure hence is inhibited from suffering the trouble that a component of the pressure-sensitive adhesive layer leaks out through or protrudes from a peripheral part of the patch. Consequently, the patch is inhibited from adhering to the inner surface of the package.

However, such a container-shape package generally includes a material having some degree of rigidity so as to retain an almost constant shape, and it is hence generally necessary to use scissors or the like for opening the package and it is difficult to open this package by hand.

### SUMMARY OF THE INVENTION

Under the circumstances described above, an object of the invention is to provide a patch package structure in which the patch is inhibited from adhering to the inner surface of the package and the package can be easily opened by hand.

In order to solve the above-mentioned object, the present invention provides the following items 1 to 10.
1. A patch package structure which comprises a package and a patch disposed in the package,
   the package comprising a first sheet material which is substantially planar and a second sheet material which has been molded,
   the second sheet material having a first region which projects outward from the package and a second region which surrounds the first region and is substantially planar,
   the first region having a planar outer shape which includes a planar outer shape of the patch, and
   the second region having a first sealed region which surrounds the first region and an unsealed region located nearer to a central part of the second sheet material than the first sealed region.
2. The patch package structure according to item 1, wherein the second sheet material has, in the unsealed region, a close-contact region in which the second sheet material is in close contact with the first sheet material.
3. The patch package structure according to item 2, wherein the close-contact region is a second sealed region.
4. The patch package structure according to item 3,
   wherein the package has an opening initiation part in a peripheral area of the package, and
   wherein the opening initiation part is located in such a position that when the package is torn at the opening initiation part, a tear is formed in the package and the tear extends to the unsealed region.
5. The patch package structure according to item 3, wherein the second sealed region has a dot-form planar shape.
6. The patch package structure according to item 3, wherein the second sealed region has a strip-form planar shape.
7. The patch package structure according to item 3, wherein the second sealed region has a substantially triangular planar shape.
8. The patch package structure according to item 3,
   wherein the boundary between the first region and the second region has a substantially rectangular shape having a first side and a second side adjoining the first side;
   the second region having the unsealed region extending along the first side; and
   the unsealed region having at least two second sealed regions.
9. The patch package structure according to item 3,
   wherein the boundary between the first region and the second region has a substantially rectangular shape having a first side and a second side adjoining the first side;
   the second region having the unsealed region extending along each of the first side and the second side; and
   the unsealed region each having at least one second sealed region.
10. The package structure according to any one of items 1 to 9,
wherein the patch is a patch preparation containing a drug.

According to the invention, since the patch is disposed in the package described above, the patch can be inhibited from receiving an external load and the patch can hence be inhibited from adhering to the inner wall of the package. In addition, since the package has a sealed region and an unsealed region as described above, the patch package structure of the invention can be opened more easily than conventional patch package structures.

### BRIEF DESCRIPTION OF THE DRAWINGS

Fig. 1A is a slant view of one embodiment of the patch package structure of the invention.
Fig. 1B is a slant view of a preferred embodiment of the patch.
Fig. 2 is a plan view of another embodiment of the patch package structure of the invention.
Fig. 3 is a plan view of still another embodiment of the patch package structure of the invention.
Fig. 4 is a plan view of a further embodiment of the patch package structure of the invention.
Fig. 5 is a plan view of still a further embodiment of the patch package structure of the invention.
Fig. 6 is a plan view of still a further embodiment of the patch package structure of the invention.
Fig. 7 is a plan view of still a further embodiment of the patch package structure of the invention.
Fig. 8 is a plan view of still a further embodiment of the patch package structure of the invention.
Fig. 9 is a slant view of still a further embodiment of the patch package structure of the invention.

### Description of Reference Numerals and Signs

- 1: patch package structure
- 2: package
- 3: patch
- 4: backing
- 5: pressure-sensitive adhesive layer
- 6: release liner
- 7: first sheet material
- 8: second sheet material
- 9: first region
- 10: second region
- 11: first sealed region
- 12: unsealed region
- 13: tear
- 14: close-contact region
- 15: second sealed region
- 16: opening initiation part

### DETAILED DESCRIPTION OF THE INVENTION

Preferred embodiments of the invention are shown below. However, the following detailed explanations thereon and specific examples are intended only for exemplification and should not be construed as limiting the scope of the invention. The following explanations on preferred embodiments are merely illustrative and are never intended to limit the invention and the applications or uses thereof.

The invention is explained in more detail by reference to the accompanying drawings, in which constituent elements are indicated by reference numerals.

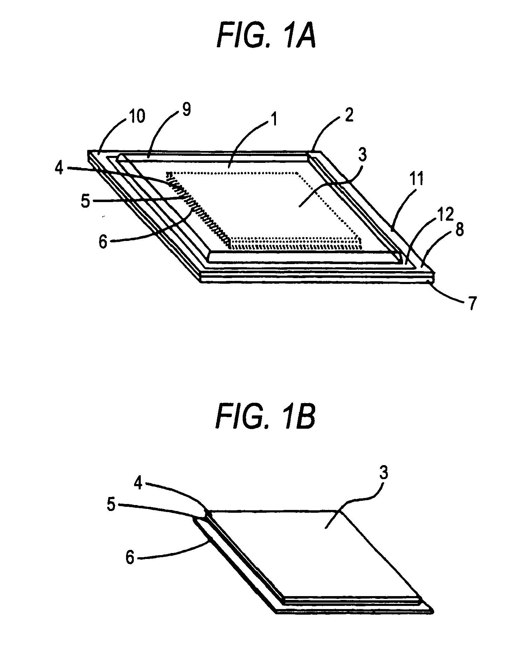

Fig. 1A is a diagrammatic slant view of one embodiment of the patch package structure 1 of the invention. This patch package structure 1 includes a package 2 and a patch 3 disposed in the package 2. The patch 3 includes a backing 4 and a pressure-sensitive adhesive layer 5 superposed on at least one side of the backing 4. It is preferred that a release liner 6 is superposed on the pressure-sensitive adhesive layer 5 in order to protect the pressure-sensitive adhesive layer 5 until the patch is used.

Fig. 1B is a diagrammatic slant view of a preferred embodiment of the patch. In this example, the release liner 6 has a planar outer shape which includes the planar outer shape of the pressure-sensitive adhesive layer 5 and that of the backing 4. The expression "one outer shape includes another outer shape" herein means not only the case where one outer shape completely includes another outer shape but also the case where part of one outer shape coincides with part of another outer shape. In this example, the periphery of the release liner 6 projects from the periphery of the pressure-sensitive adhesive layer 5 and that of backing 4. This inhibits the periphery of the pressure-sensitive adhesive layer 5 from coming into contact with the inner wall of the packing 2 even when the patch 3 moves in the package 2. Because of this, the patch 3 is less apt to adhere to the inner wall of the package 2.

Reference is made to Fig. 1A again. The package 2 includes a first sheet material 7 which is substantially planar and a second sheet material 8 which has been molded. The second sheet material 8 has a first region 9 which projects outward from the package 2 to form a projecting part, and further has a second region 10 which surrounds the first region 9 and is substantially planar. The second region 10 has a first sealed region 11 and an unsealed region 12. The first region 9 has a planar outer shape including the planar outer shape of the patch 3. Since the second sheet material 8 has such a shape, the patch 3, in particular, the periphery thereof, is less apt to receive an external load although it may come into contact with the inner wall of the package 2. Therefore, a component of the pressure-sensitive adhesive layer is less apt to protrude from a peripheral part of the patch 3. In case where a component of the pressure-sensitive adhesive layer has protruded, this component is less apt to adhere to the inner wall of the package 2.

As used herein, the phrase "a component of a pressure-sensitive adhesive layer" is meant to encompass not only one component of a pressure-sensitive adhesive layer but also a plurality of components and a composition thereof.

In Fig. 1A and Fig. 1B, the package and the patch have been drawn so that the planar outer shapes thereof are substantially rectangular (they may be square; the same applies hereinafter). However, the planar outer shapes thereof are not limited to substantially rectangular ones, and examples thereof include substantially elliptic shapes, substantially circular shapes, substantially polygonal shapes such as substantially triangular shapes, substantially quadrangular shapes and substantially pentagonal shapes, and other various shapes. From the standpoint of the efficiency of material utilization, substantially rectangular shapes are preferred. Although the mode in which the patch is disposed in the package is not particularly limited, it is preferred that the patch is disposed so that the release liner 6 directly faces the inner wall of the first sheet material 7, as shown in Fig. 1A. This is because disposition in this mode is effective in preventing the backing 4, which is flexible, from receiving an impact from outside the package 2 because the second sheet material 8 has been molded into a given shape.

The sizes of the patch and package vary depending on the intended use of the patch. However, in the case where they have a substantially rectangular planar outer shape, the sizes thereof in a specific example are as follows. In a specific example, the patch has a substantially rectangular shape in which a first side has a length of about 20-100 mm and a second side has a length of about 20-100 mm, and has a thickness of about 30-400 µm. In this specific example, the package has a substantially rectangular shape in which a first side has a length of about 25-130 mm and a second side has a length of about 25-130 mm. In this specific example, the first region of the package has a substantially rectangular shape in which a first side has a length of about 21-110 mm and a second side has a length of about 21-110 mm. In this specific example, the second region of the package has a strip shape having a width of about 2-10 mm. In this specific example, the first region projects to a height of about 0.5-2.0 mm from the second region.

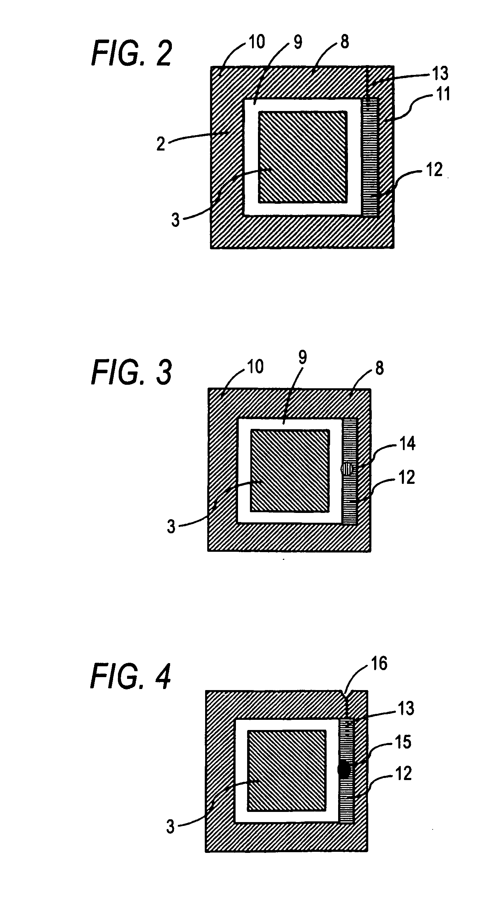

In the next place, Fig. 2 is a diagrammatic plan view of another embodiment of the patch package structure of the invention which is viewed from the second sheet material 8 side. This embodiment has the same constitution as the embodiment shown in Fig. 1, except the matters specially mentioned. The patch 3 is located in the first region 9. The second region 10 has a first sealed region 11 which surrounds the first region 9 and further has at least one unsealed region 12 located nearer to a central part of the second sheet material than the first sealed region 11. From the standpoint of easy opening, it is preferred that a part of the outer shape of the unsealed region 12 which is located on the side facing a central part of the package 2 is near to the boundary between the first region 9 and the second region 10 or be located substantially on the boundary.

Techniques for obtaining the sealed region 11 are not particularly limited so long as the first sheet material and the second sheet material can be sealed to each other. For example, fusion bonding such as heat sealing or adhesive bonding can be used to obtain the sealed region 11. On the other hand, in the unsealed region 12, the first sheet material and the second sheet material are not sealed to each other. The first and second sheet materials may be in contact with each other or apart from each other, and need not be in close contact with each other. It is preferred that in the unsealed region, the first sheet material and the second sheet material are in close contact with each other to form one or more close-contact regions. Although the unsealed region in Fig. 2 is drawn so as to have a substantial strip form, the shape thereof should not be construed as being limited to strip forms. In the case of the specific example described above, the width of the unsealed region is, for example, 1.5-5 mm. The term "close contact" herein means that the distance between the first sheet material and the second sheet material is shorter than the thickness of the patch.

With Reference to Fig. 2, the patch package structure of the invention is opened, for example, in the following manner. The user tears any desired part of the periphery of the package with fingers or another means to form a tear 13 in the package, which is indicated by a broken line in the figure. For sufficiently producing an effect of the invention, it is desirable that the tear 13 extends to the unsealed region 12. When the tear 13 extends to the unsealed region 12, the sections of the tear 13 include an opening where the first sheet material is not sealed to the second sheet material in the unsealed region 12. It is preferred that the package is opened at this time. In case where the opening is small, this opening may be enlarged with fingers optionally inserted into the opening. The package is thus opened, and the patch 3 can be taken out.

Fig. 3 is a diagrammatic plan view of still another embodiment of the patch package structure of the invention which is viewed from the second sheet material 8 side. This embodiment has the same constitution as the embodiments described above, except the matters specially mentioned. This embodiment has at least one close-contact region 14 in the unseated region 12. The package may have only one close-contact region 14. It is, however, preferred to dispose two or more close-contact regions 14 from the standpoint of sufficiently producing an effect of the invention.

The close-contact region 14 need not be a region where the first sheet material has been sealed to the second sheet material, so long as the first and second sheet materials are sufficiently near to each other. In the close-contact region, the distance between the inner wall of the first sheet material and the inner wall of the second sheet material is shorter than the thickness of the patch 3. Owing to this close-contact region, a peripheral part of the patch is inhibited from being caught in the gap between the first sheet material and second sheet material even when the patch moves in the package. Because of this, the possibility that the patch might be torn together with the package upon opening is reduced. Techniques for obtaining the close-contact region are not particularly limited. Examples thereof include a method in which the first sheet material and/or the second sheet material in the region are slightly projected toward each other. In this embodiment, the close-contact region 14 has been drawn so as to have an elliptic planar shape. However, the planar shape of the close-contact region 14 should not be construed as being limited to elliptic ones. Other examples thereof will be described later.

In the next place, reference is made to Fig. 3. In this embodiment, the close-contact region 14 is disposed so as to be near to the first region 9. It is preferred that a part of the periphery of the close-contact region 14 which is located nearest to a central part of the package is located substantially on the boundary between the first region 9 and the second region 10, as shown in this figure. Owing to this constitution, even when the patch 3 moves in the package, the close-contact region 14 functions as a stopper for the periphery of the patch 3. As a result, a peripheral part of the patch 3 is efficiently inhibited from being caught in the gap between the first sheet material and second sheet material in the unsealed region 12.

Fig. 4 is a diagrammatic plan view of a further embodiment of the patch package structure of the invention which is viewed from the second sheet material side. This embodiment has the same constitution as the embodiments described above, except the matters specially mentioned. As shown in the figure, in this embodiment, the close-contact region has a sealed region formed therein. Hereinafter, this region is referred to as a second sealed region 15. This is because in order for the close-contact region to sufficiently perform its function, it is preferred to seal the first and second sheet materials to each other.

In this embodiment, the package has one or more opening initiation parts 16 in a peripheral area of the package. The opening initiation part 16 in this embodiment has been drawn as a substantially V-shaped notch in the package. The planar shape of the notch should not be construed as being limited to that shape, and may be a substantially quadrangular shape such as a substantial 1-shape or a substantially trapezoidal shape or a substantially polygonal shape such as a substantially pentagonal shape. This constitution enables the user to easily tear the package at the opening initiation part 16. The opening initiation part is disposed so as to result in a tear 13 extending to the unsealed region 12.

The direction in which a tear is to be formed is governed by the planar shape of the notch. For example, in the case of a substantially V-shaped notch, a tear will be formed along the extension of a straight line which nearly bisects the V-shaped vertex angle, unless the user tears the package with special intention. Alternatively, in the case of a substantially I-shaped notch, a tear will be likewise formed along the extension of the shape I. Consequently, a notch is disposed in such a position that the unsealed region is present on such an extension of the straight line. In addition, the shape of the tear to be formed can be shown as an illustration on the outer wall of the package, or how to tear the package can be described in the directions to be attached to the patch package structure.

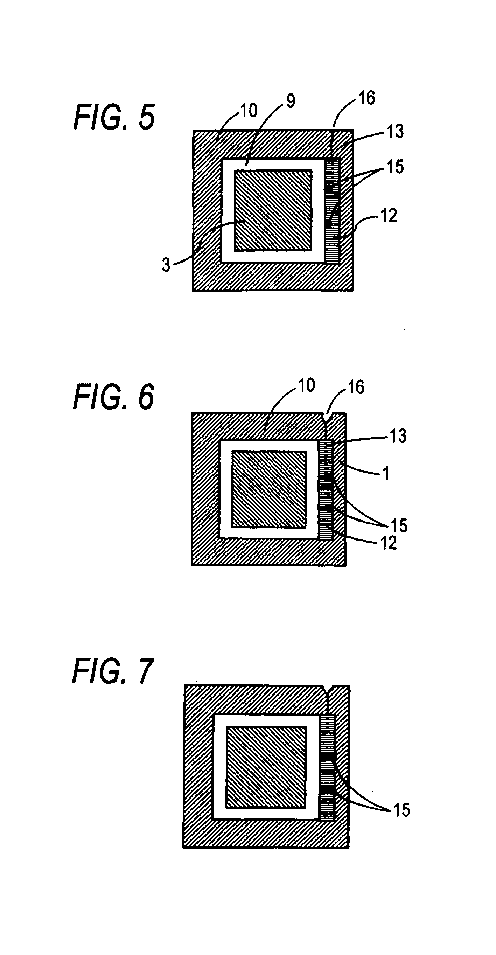

Fig. 5 is a diagrammatic plan view of still a further embodiment of the patch package structure of the invention which is viewed from the second sheet material side. This embodiment has the same constitution as the embodiments described above, except the matters specially mentioned. This embodiment has an opening initiation part 16 which is a substantially I-shaped notch. In this embodiment, the second region 10 has at least one strip-form unsealed region 12. In this embodiment, each unsealed region has at least one, preferably two or more second sealed regions 15. In this embodiment, the boundary between the first region 9 and the second region 10 has a substantially rectangular shape, and the package has at least one, preferably two or more second sealed regions 15 along one side of the substantially rectangular shape. Due to the constitution in which the package has two or more second sealed regions along one side of such a substantially rectangular shape, the patch 3 is efficiently inhibited from being caught in the gap between the first sheet material and second sheet material in the unsealed region 12.

In this embodiment, the second sealed regions 15 have a dot-form planar shape.

It is preferred in the invention that the planar outer shape of each second sealed region 15 is small. For example, in the case where the strip-form unsealed region 12 is split into a package-center-side half and a package-periphery-side half, the planar outer shape of each second sealed region 15 is included in the package-center-side half. When the user tears the package to form a tear 13, a lower force suffices for the user to tear the region which has not been sealed (i.e., the unsealed region 12) than in the case of tearing the region which have been sealed (i.e., the second sealed region 15). Consequently, the second sealed region 15 having a small planar outer shape is preferred because the user can easily form a tear in the package.

Furthermore, in the case that the user tears the package to form a tear 13 and the user then peels off the first and second sheet materials in the second sealed region 15 in the resultant opening, a lower peeling force suffices when the second sealed region 15 has a smaller area. The dot-form second sealed region 15 is preferred from the standpoint of ease of production.

Fig. 6 is a diagrammatic plan view of still a further embodiment of the patch package structure of the invention which is viewed from the second sheet material side. This embodiment has the same constitution as the embodiments described above, except the matters specially mentioned. This embodiment has an opening initiation part 16 which is a substantially trapezoidal notch. This embodiment has second sealed regions 15 each having a substantially triangular shape. Two vertexes of each substantially triangular shape are located near to the boundary between the first sealed region 11 and the unsealed region 12, and preferably located substantially on the boundary. The remaining vertex of each substantially triangular shape is located near to the boundary between the first region and the second region, and preferably located substantially on the boundary. In this embodiment, when a tear 13 is formed, second sealed region 15 is divided by the tear 13 into two portions, and the package-center-side portion has a smaller area than the package-periphery-side portion. Owing to this constitution, the user can easily peel off the first and second sheet materials in the center-side portions of the second sealed regions 15 of the package to open the package.

Fig. 7 is a diagrammatic plan view of still a further embodiment of the patch package structure of the invention which is viewed from the second sheet material side. This embodiment has the same constitution as the embodiments described above, except the matters specially mentioned. This embodiment has an opening initiation part 16 which is a substantially V-shaped notch. This embodiment has second sealed regions 15 which each have a strip-form planar shape extending in a direction perpendicular to the nearest side of the package. This planar shape attains high sealing strength, so that it contributes to an improvement in the hermetic sealing of the package.

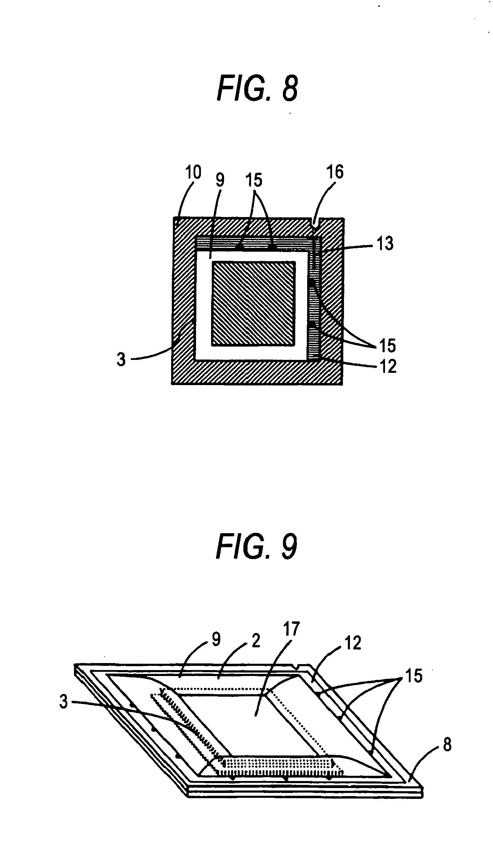

Fig. 8 is a diagrammatic plan view of still a further embodiment of the patch package structure of the invention which is viewed from the second sheet material side. This embodiment has the same constitution as the embodiments described above, except the matters specially mentioned. This embodiment has an opening initiation part 16 which is a substantially pentagonal notch. In this embodiment, the package has a substantially rectangular planar shape having a first side and a second side adjoining the first side. In this embodiment, the boundary between the first region 9 and the second region 10 has a substantially rectangular shape having a first side and a second side adjoining the first side, and the second region 10 has an unsealed region 12 extending along each of the first side and the second side. The unsealed region 12 has second sealed regions 15 each having a dot-form planar outer shape, which have been disposed along the first side and second side. This package may have an unsealed region and second sealed regions along another side.

This constitution has the following advantage when the package is torn at the opening initiation part 16 to form a tear 13 and open the package. Namely, the first sheet material and the second sheet material can be easily peeled off from an area around the vertex located between the first side and second side of the substantially rectangular boundary between the first region 9 and the second region 10.

Fig. 9 is a diagrammatic slant view of still a further embodiment of the patch package structure of the invention. This embodiment has the same constitution as the embodiments described above, except the matters specially mentioned. In this embodiment, the second sheet material 8 has been molded into a given shape in the first region 9. This shape in the first region 9 includes a protrudent part projecting outward from the package 2, and the protrudent part includes, in a nearly central part thereof, a recessed part 17 which is depressed toward the inside of the package 2. This recessed part 17 inhibits the patch 3 from moving in the thickness direction within the package 2. In addition, since the space within the package has a reduced volume, the patch has improved stability. Furthermore, owing to this recessed part 17, the periphery of the patch, in which the release liner has an outer shape including the outer shape of the pressure-sensitive adhesive layer and that of backing as in the patch shown in Fig. 1B, is less apt to coming into contact with the inner wall of the package. Consequently, a component of the pressure-sensitive adhesive layer is less apt to adhere to the inner wall of the package.

With reference to Fig. 9, a substantially strip-form unsealed region 12 is disposed along each side of the substantially rectangular package 2. Each unsealed region 12 has at least one, preferably two or more, e.g., two or three, dot-form second sealed regions 15. The second sealed regions 15 are disposed in such positions that when each strip-form unsealed region 12 is split into a package-center-side half and a package-periphery-side half, then the second sealed regions 15 are included in the package-center-side half.

The first sheet material and second sheet material in the patch package structure of the invention described above are not particularly limited so long as both materials can be sealed together for forming the package. Heat-sealable sheet materials are preferred from the standpoint of ease of production. Examples of such packaging materials include films of resins such as polyolefins including polyethylene and polypropylene, polyesters including poly(ethylene terephthalate), and other resins including poly(vinyl chloride) and polyacrylonitrile, metal films such as aluminum foils, materials obtained by vapor-depositing aluminum on these films, and laminated films obtained by laminating two or more thereof.

From the standpoints of impermeability to package contents such as a drug and heat sealability, a polyacrylonitrile film or the like is preferred for use as such a packaging material. From the standpoint of the property of not adsorbing package contents such as a drug, it is preferred to employ a polyester, in particular, poly(ethylene terephthalate) or the like. From the standpoint of the property of being impermeable to or not transmitting package contents (components of the patch and the gas in the package), light rays, or gases, more preferred packaging materials are those resin films which have undergone aluminum vapor deposition and laminated films obtained by laminating an aluminum foil to those resin films. More preferred from the standpoint of reconciling those properties are laminated films obtained by laminating a polyester, in particular poly(ethylene terephthalate), with a polyacrylonitrile film. Most preferred is a laminated film obtained by laminating a polyester, in particular poly(ethylene terephthalate), with an aluminum foil or vapor-deposited aluminum layer and a polyacrylonitrile film. From the standpoint of the storage stability of package contents such as a drug, a laminated film obtained by laminating a water-impermeable layer and a water-permeable layer respectively to the outer side and inner side of a hygroscopic layer containing a drying agent is also preferred.

Materials and constitutions of the first sheet material and the second sheet material may be the same or different. In the case where the second sheet material to be used is a molded sheet material, it is preferably made of a rigid material because the molded sheet material is required to retain a given shape. In the case where the first sheet material is used as an approximately planar unmolded sheet material without being molded, the first sheet material is preferably made of a flexible material because such unmolded sheet material can be easily sealed to the second sheet material to thereby facilitate production.

The thickness of the first sheet material is not particularly limited. However, it is preferably 10-200 µm, more preferably 20-100 µm, from the standpoints of production efficiency and impermeability to ingredients to be packaged in the package structure.

The thickness of the second sheet material is not particularly limited. It is, however, preferred that the second sheet material have some degree of stiffness because of the necessity of retaining the given shape. From this standpoint, the thickness thereof is preferably 50-300 µm, more preferably 50-200 µm.

Molding methods for obtaining the molded sheet material having the given shape are not limited. Examples thereof include vacuum/pressure molding, injection molding, and press molding. From the standpoints of suitability for cost reduction, degree of freedom of shapes, material selection, etc., vacuum molding, pressure molding, and the like are preferred.

The patch may be a patch preparation in which the pressure-sensitive adhesive layer contains a drug. The drug herein is not particularly limited. Preferred is a drug which can be administered to a mammal such as a human being through the skin, i.e., which is percutaneously absorbable. Examples of such drugs include systemic anesthetics, hypnotic agents, antiepileptics, antipyretic/analgesic/antiphlogistic agents, antidizzying agents, psychoneurotics, local anesthetics, skeletal muscle relaxants, agents for autonomous nerve, antispasmodics, anti-Parkinsonian agents, antihistamines, cardiotonics, antiarrhythmics, diuretics, antihypertensives, vasoconstrictors, coronary vasodilators, peripheral vasodilators, antiarteriosclerotic agents, agents for circulatory organs, respiration facilitators, antitussive/expectorant agents, hormone drugs, external-use preparations for purulent diseases, analgesic/antipruritic/astringent/antiphlogistic agents, agents for parasitic skin diseases, hemostats, antipodagrics, agents for diabetes, antineoplastics, antibiotics, chemotherapeutics, narcotics, and smoking renunciation aids.

The content of the percutaneously absorbable drug is not particularly limited so long as it sufficiently produces the effect thereof and does not impair the adhesiveness of the pressure-sensitive adhesive. However, the content thereof in the pressure-sensitive adhesive is, for example, 0.01-70% by weight, preferably 0.1-60% by weight, more preferably 0.5-40% by weight. In case where the content thereof is lower than 0.01 % by weight, there is a possibility that the remedial effect might be insufficient. In case where the content thereof is higher than 70% by weight, there is a possibility that skin irritation might occur and such a large drug amount might be economically disadvantageous.

The pressure-sensitive adhesive layer contains a pressure-sensitive adhesive. The pressure-sensitive adhesive is not particularly limited. Examples thereof include acrylic pressure-sensitive adhesives containing an acrylic polymer; rubber pressure-sensitive adhesives such as styrene/diene/styrene block copolymers (e.g., styrene/isoprene/styrene block copolymers and styrene/butadiene/styrene block copolymers), polyisoprene, polyisobutylene, and polybutadiene; silicone pressure-sensitive adhesives such as silicone rubbers, dimethylsiloxane-based polymers, and diphenylsiloxane-based polymers; vinyl ether pressure-sensitive adhesives such as poly(vinyl methyl ether), poly(vinyl ethyl ether), and poly(vinyl isobutyl ether); vinyl ester pressure-sensitive adhesives such as vinyl acetate/ethylene copolymers; and polyester pressure-sensitive adhesives produced from a carboxylic acid ingredient such as dimethyl terephthalate, dimethyl isophthalate, or dimethyl phthalate and a polyhydric alcohol ingredient such as ethylene glycol.

Acrylic pressure-sensitive adhesives or rubber pressure-sensitive adhesives are preferred among such pressure-sensitive adhesives because acrylic or rubber pressure-sensitive adhesives give a pressure-sensitive adhesive layer which is capable of holding a liquid component therein and hence can give a soft feeling during wear on the skin. In particular, acrylic pressure-sensitive adhesives are preferred because they can be easily crosslinked and give a pressure-sensitive adhesive layer capable of holding a large amount of a liquid component therein.

Examples of such acrylic pressure-sensitive adhesives include acrylic ester pressure-sensitive adhesives containing as the main component a polymer comprising monomer units derived from one or more C₂₋₁₈ alkyl esters of (meth)acrylic acid. Examples of those rubber pressure-sensitive adhesives include rubber pressure-sensitive adhesives containing as the main component at least one member selected from polyisobutylene, polyisoprene, and styrene/diene/styrene copolymers.

The liquid component is not particularly limited. From the standpoint of compatibility with the pressure-sensitive adhesive layer, organic liquid ingredients are preferred. Although the organic liquid ingredients are not particularly limited, ones having the effect of accelerating percutaneous absorption are preferred. Examples of such organic liquid ingredients include glycols such as ethylene glycol, diethylene glycol, propylene glycol, triethylene glycol, polyethylene glycol, and polypropylene glycol; fats and oils such as olive oil, caster oil, squalane, and lanolin; hydrocarbons such as liquid paraffin; various surfactants; ethoxy stearyl alcohol; glycerol monoesters such as oleic acid monoglyceride, caprylic acid monoglyceride, and lauric acid monoglyceride, glycerol diesters, glycerol triesters, and mixtures thereof; alkyl esters of fatty acids, such as ethyl laurate, isopropyl myristate, isotridecyl myristate, octyl palmitate, isopropyl palmitate, ethyl oleate, and diisopropyl adipate; higher fatty acids such as oleic acid and caprylic acid; and other compounds including N-methylpyrrolidone and 1,3-butanediol.

In the case where a liquid component is contained in the pressure-sensitive adhesive layer, there is a possibility that during storage of the patch, the pressure-sensitive adhesive layer might plasticize and components of the pressure-sensitive adhesive layer might protrude or flow out from the periphery of the patch. The invention is advantageously practiced especially in such cases. From this standpoint, the content of the liquid component in the pressure-sensitive adhesive layer is preferably 5-70% by weight, more preferably 10-65% by weight, most preferably 15-60% by weight.

When the pressure-sensitive adhesive layer is relatively thick, the protrusion or outflow of the pressure-sensitive adhesive layer from the periphery of the patch tends to occur. The invention is advantageously practiced especially in such cases. From this standpoint, the thickness of this pressure-sensitive adhesive layer is preferably 20-300 µm, more preferably 30-250 µm, most preferably 50-200 µm.

The explanations of the invention given above are merely illustrative, and modified embodiments thereof which do not depart from the spirit of the invention are hence intended to be within the scope of the invention. Such modified embodiments should not be construed ad departing from the spirit and scope of the invention.

The present application is based on Japanese Patent Application No. 2008-026631 filed on February 6, 2008, and the contents are incorporated herein by reference.

## Claims

1. A patch package structure which comprises a package and a patch disposed in the package,
the package comprising a first sheet material which is substantially planar and a second sheet material which has been molded,
the second sheet material having a first region which projects outward from the package and a second region which surrounds the first region and is substantially planar,
the first region having a planar outer shape which includes a planar outer shape of the patch, and
the second region having a first sealed region which surrounds the first region and an unsealed region located nearer to a central part of the second sheet material than the first sealed region.

2. The patch package structure according to claim 1, wherein the second sheet material has, in the unsealed region, a close-contact region in which the second sheet material is in close contact with the first sheet material.

3. The patch package structure according to claim 2, wherein the close-contact region is a second sealed region.

4. The patch package structure according to claim 3,
wherein the package has an opening initiation part in a peripheral area of the package, and
wherein the opening initiation part is located in such a position that when the package is torn at the opening initiation part, a tear is formed in the package and the tear extends to the unsealed region.

5. The patch package structure according to claim 3, wherein the second sealed region has a dot-form planar shape.

6. The patch package structure according to claim 3, wherein the second sealed region has a strip-form planar shape.

7. The patch package structure according to claim 3, wherein the second sealed region has a substantially triangular planar shape.

8. The patch package structure according to claim 3,
wherein the boundary between the first region and the second region has a substantially rectangular shape having a first side and a second side adjoining the first side;
the second region having the unsealed region extending along the first side; and
the unsealed region having at least two second sealed regions.

9. The patch package structure according to claim 3,
wherein the boundary between the first region and the second region has a substantially rectangular shape having a first side and a second side adjoining the first side;
the second region having the unsealed region extending along each of the first side and the second side; and
the unsealed region each having at least one second sealed region.

10. The package structure according to any one of claims 1 to 9,
wherein the patch is a patch preparation containing a drug.
